# EUROPÄISCHE PATENTSCHRIFT

(11) **EP 2 644 183 B1**
(45) Veröffentlichungstag und Bekanntmachung des Hinweises auf die Patenterteilung: **16.12.2020**
(21) Anmeldenummer: 13158314.8
(22) Anmeldetag: 08.03.2013
(51) Int. Cl.: A61K 6/60, A61K 6/71, A61K 6/77

(54) **Polymerisierbare Dentalkomposite mit verbesserten Gebrauchseigenschaften, Verfahren zur Einstellung der Gebrauchseigenschaften polymerisierbarer Dentalkompositen, und nach diesen Verfahren optimierte Dentalkomposite**
Polymerisable dental composites with improved usage properties, method for adjusting the usage properties of polymerisable dental composites and dental composites optimised using these methods
Composite dentaire polymérisable doté de propriétés d'utilisation améliorées, procédé de réglage des propriétés d'utilisation de composites dentaires polymérisables et composite dentaire optimisé selon ce procédé

(30) Priorität: 28.03.2012 DE 102012006152
(43) Veröffentlichungstag der Anmeldung: 02.10.2013
(73) Patentinhaber: Kulzer GmbH, 63450 Hanau (DE)
(72) Erfinder: Dr. Utterodt, Andreas, 61267 Neu-Anspach (DE); Reischl, Kurt, 35799 Merenberg (DE); Schönhof, Nelli, 35619 Braunfels (DE); Eck, Michael, 61389 Schmitten (DE); Hiersekorn, Christine, 65599 Dornburg (DE); Schneider, Jutta, 65594 Runkel (DE)
(74) Vertreter: Bendele, Tanja

(56) Entgegenhaltungen:
- EP-A1- 1 502 571
- EP-A1- 1 872 767
- EP-A2- 2 193 776
- WO-A1-2006/111373
- ERTL K ET AL: "Stickiness of dental resin composite materials to steel, dentin and bonded dentin", DENTAL MATERIALS, ELSEVIER, AMSTERDAM, NL, vol. 26, no. 1, 1 January 2010 (2010-01-01), pages 59-66, XP026799078, ISSN: 0109-5641 [retrieved on 2009-10-17]
- Schott AG: "Shott dental glass inert - Materials Data", , 30 April 2012 (2012-04-30), Retrieved from the Internet: URL:https://www.schott.com/dentalglass [retrieved on 2020-12-31]
- Ernst Lautenbach: "Wörterbuch für Zahnmedizin", 1 February 1992 (1992-02-01) * p. 821-822, 1535-1536 *

## Beschreibung

Die Erfindung betrifft ein neuartiges Verfahren zur Einstellung der Gebrauchseigenschaften von polymerisierbaren fließfähigen, in der Regel ungehärteten Dentalkompositen, Dentalkomposite mit verbesserten oder optimierten Gebrauchseigenschaften, insbesondere nach dem Verfahren hergestellte Dentalkomposite.

Nach dem Stand der Technik sind viele Dentalkomposite bekannt, die universell einsetzbar sind, d.h. die Anforderungen für die Restauration von Zahnhartsubstanz in allen Kavitätenklassen I, II, III, IV und V nach G. V. BLACK erfüllen. Aus der Materialklasse der Dentalkomposite eignen sich dafür grundsätzlich nur die anorganisch-organischen Hybridmaterialien mit größeren Anteilen anorganischer Füllkörper wie z.B. Dentalglas oder mineralischen nano-Agglomeraten. Die in den 80er Jahren eingeführten Microfüllerkomposite mit Präpolymerfüllern eignen sich aufgrund der limitierten Verschleißfestigkeit (Abrasionsfestigkeit) und Bruchfestigkeit (Biegebruchfestigkeit) nicht für den Einsatz im Seitenzahn (Klassen I und II). Vorteilhaft ist ein hoher Füllstoffgehalt, um sehr gute mechanische Eigenschaften des ausgehärteten Komposites zu erreichen und gleichzeitig den auftretenden Polymerisationschrumpf bei der Aushärtung zu reduzieren. Diese Eigenschaften sind auch maßgeblich für den Langzeiterfolg der Behandlungstherapie mit Dentalkompositen.

EP2193776A2 offenbart Dental-Kompositmaterialien auf (Meth)acrylatbasis mit anorganischen Füllstoffen. EP1872767A1 offenbart ein pastöses Dentalmaterial enthalten ein PEG 400 (PEG mit 400 g/mol). WO2006/111373A1 betrifft ebenfalls Dentalmaterialien mit anorganischen Füllstoffen und einem Urethan-Gruppen enthaltenden Silan. EP1502571A1 offenbart Dentalmaterialien umfassend Splitterpolymere und anorganisch verstärkte Perlpolymere.

Neben den Materialeigenschaften eines ausgehärteten Dentalkomposits ist aber auch die Verarbeitung vor der Polymerisation durch den Anwender entscheidend für den Langzeiterfolg der medizinischen Versorgung. Das Applizieren aus dem Packmittel, Einbringen in die Kavität, Adaptieren an die Zahnhartsubstanz und Modellieren der Kompositschicht ist dabei extrem abhängig von der Plastizität und Klebrigkeit des unpolymerisierten Materials. Gerade die gewünschten hohen Füllstoffanteile anorganischer Füller bringen wegen der Oberfächenwechselwirkung eine nachteilige Thixotropie in das System. Verursacht durch die polaren Wechselwirkungen unter den oxydischen Füllstoffpartikeln (Silikate) können diese Kräfte unter Scherung zum Teil temporär gestört werden, und die Komposite sind durch eine Strukturviskosität charakterisiert. Die gleichen Wechselwirkungen führen an der Oberfläche des Komposites zur Klebrigkeit. Insbesondere weiche Komposite neigen wegen des besseren Fließverhaltens zu einer nachteiligen Klebrigkeit.

Zur Bestimmung des viskoelastischen Fließverhaltens werden für gewöhnlich Rheometer eingesetzt. Nach heutigem Stand der Technik wird die Konsistenz der Komposite allein durch geringfügige Anpassung des Füllstoffanteils korrigiert. Dabei verändert sich gleichzeitig die Klebrigkeit unkontrolliert. Es ist bis heute nicht üblich, die Klebrigkeit der Komposite überhaupt zu messen bzw. kontrolliert einzustellen. Die Produkteigenschaften variieren deshalb in einem größeren Bereich, so dass die Qualitätsvarianz durch den Anwender wahrnehmbar ist.

### Aufgabe

Für eine möglichst anwendungssichere und erfolgreiche zahnärztliche Versorgung oder zahntechnische Arbeit mit fließfähigen Dentalkompositen werden als Gebrauchs- bzw. Handlingseigenschaften eine nicht zu feste Plastizität (ausreichendes Fließverhalten) und eine geringe Klebrigkeit (am Applikationsinstrument) gewünscht.

### Die Erfindung

Die Erfindung betrifft ein Verfahren zur Einstellung oder Verbesserung oder Optimierung der Gebrauchseigenschaften von Dentalkompositen, besonders solcher mit hohem Füllstoffanteil, durch Inbeziehungsetzen und Optimieren der Parameter Texturierung und Klebrigkeit. Die Erfindung betrifft außerdem Dentalkomposite, insbesondere nach dem Verfahren optimierte Dentalkomposite, die vor allem in Bezug auf die Parameter Texturierung und Klebrigkeit verbesserte Gebrauchseigenschaften aufweisen. Insbesondere werden die weiter unten beschriebenen Komponenten A1 und A2 als Additive oder Füllstoffanteil in den Dentalkompositen eingesetzt.

Nach dem Verfahren der Erfindung eingestellte bzw. optimierte Dentalkomposite weisen durch ihre spezielle Zusammensetzung eine optimale Balance von Plastizität und Klebrigkeit bei gleichzeitig sehr guten Materialeigenschaften auf. Hervorragenden Materialeigenschaften, insbesondere die niedrigere Schrumpfkraft bei hoher Biegefestigkeit, sind von Kompositen basierend auf dem Vernetzer TCD-di-HEA beschrieben (EP 1 719 497 A1, EP 2 193 776 A2). Die Urethan-Vernetzer (TCD-di-HEA, HEMA-TMDI) bilden üblicherweise Wasserstoffbrücken aus und führen damit zu einer stärkeren Klebrigkeit bzw. festen Konsistenz/Plastizität. Eine vorzugsweise weiche Plastizität führt in derartigen Systemen automatisch zu einer unerwünscht starken Klebrigkeit.
Zur Lösung der gestellten Aufgabe ist auf der stofflichen Seite das Einbringen von zwei zusätzlichen Komponenten A1 und A2 in kleineren Anteilen bis jeweils maximal 5 Gew.-%, bevorzugt von 0,5 bis 5, besonders bevorzugt von 1 bis 4 Gew. -% , von Vorteil.

### Komponente A1: Prä-Polymer-Füller

Bei Zusatz von Prä-Polymer-Füllern, auch als Splitterpolymere oder Splitterpolymerisate bekannt, ist nach dem Fachwissen zu erwarten, dass die Strukturviskosität geringer wird. Neben diesem bekannten Effekt wurde jedoch von den Erfindern eine überraschend deutlich geringere Klebrigkeit an der Kompositoberfläche festgestellt (wie sie durch Messen der Abzugskraft quantifiziert werden kann, wie weiter unten beschrieben). Durch Variation des vergleichsweise geringen Anteils der Komponente A1 kann die Klebrigkeit in einem weiten Bereich eingestellt werden, ohne stärkere Veränderungen der Strukturviskosität zu bewirken. Die mechanischen Eigenschaften bleiben überraschend stabil, obgleich die Polymerpartikel deutlich weicher sind.

### Komponente A2: hydroxyfunktionalisierte Additive

Überraschend vorteilhaft wirkt sich auch die Zugabe von flüssigen hydroxyfunktionalisierten Additiven mit einem Molekulargewicht < 250 g/mol, bevorzugt entsprechend der weiter unten angegebenen Strukturformel (I), auf die Senkung der Plastizität (das entspricht einer Verringerung der Strukturviskosität) aus. Diese Wirkung ist überraschend, weil polare hydroxyfunktionalisierte Substanzen dafür bekannt sind, die Wechselwirkungen und damit die Thixotropie zu erhöhen. Ohne die Erfindung durch eine Theorie zu beschränken, lässt sich der gefundene Effekt eventuell damit erklären, dass kleine Moleküle die bestehenden Wechselwirkungen auch reduzieren können, indem sie vermutlich zwischen die vorhandenen polaren Gruppen eindringen und damit eine direkte Wechselwirkung der Partikeloberflächen reduzieren.

Als derartige Moleküle mit geringem Molekulargewicht lassen sich diejenigen der folgenden Formel

H-Y-Z (I)

nennen, worin bedeuten:
Y = -O-, -S-, -CO-, -OSi(OR¹)₂-, -OE
Z = H, OH, SH, NH₂, COOH, COOR²
E = -CₙHₘOp-
R¹ = H, C₁-C₄-Alkyl
R² = C₁-C₁₅-Alkyl, durch ein oder mehrere O-Atome unterbrochenes C₂-C₁₄-Alkyl,
n = 2 bis 5
m = 4 bis 11
p = n.

C₁-C₁₅-Alkyl kann linear oder verzweigt sein und bedeutet beispielsweise Methyl, Ethyl, Propyl, Isopropyl, Butyl, Isobutyl, sec.-Butyl, tert.-Butyl, Pentyl, Isopentyl, Hexyl, Heptyl, Octyl, Nonyl, Decyl, Dodecyl oder Pentadecyl. Bevorzugt sind C₁-C₁₂-, z.B. C₁-C₈- oder C₁-C₆-, insbesondere C₁-C₄-Alkyl. C₁-C₁₂-Alkyl, C₁-C₈-Alkyl, C₁-C₆Alkyl und C₁-C₄-Alkyl können die gleichen Bedeutungen haben wie oben angegeben, bis zur entsprechenden Anzahl der C-Atome.
Ein durch ein oder mehrere O-Atome unterbrochenes C₂-C₁₄-Alkyl ist beispielsweise 1 bis 5, z.B. 1 bis 3 oder 1 oder 2 mal durch -O- unterbrochen. Es ergeben sich z.B. Struktureinheiten wie-O(CH₂)₂OH,-O(CH₂)₂OCH₃,-O(CH₂CH₂O)₂CH₂CH₃,-CH₂-O-CH₃,-CH₂CH₂-O-CH₂CH₃,-[CH₂CH₂O]_{y}-CH₃, mit y = 1 bis 5, -(CH₂CH₂O)₅CH₂CH₃, -CH₂-CH(CH₃)-O-CH₂-CH₂CH₃ oder -CH₂-CH(CH₃)-O-CH₂-CH₃.
Beispiele für bevorzugte derartige Verbindungen mit geringem Molekulargewicht von unter 250 g/mol sind neben Wasser niedermolekulare Alkohole wie Ethanol, Propanol, Isopropanol, n-Butanol, mehrwertige Alkohole, z.B. Glykole wie Ethylenglykol, Glycerin oder Polyethylenglykole, aber auch methacrylfunktionelle Silane,

Besonders bevorzugt sind Ethanol, Glycerin, Polyethylenglykol 200 und methacrylfunktionelle Silane wie z.B. das als "MEMO" oder "Silan A174" bekannte gamma-(Methacryloxy)-propyl-trimethoxysilan (CAS-Nummer: 2530-85-0), sowie Wasser.

Mit dem erfindungsgemäßen Verfahren kann man die Eigenschaften vorhandener Rezepturen einstellen; es kann aber auch bei der Entwicklung neuer Rezepturen eingesetzt werden. Zweckmäßig geht man von bekannten Dentalkompositen mit hohem Füllstoffanteil aus. Ziel des erfindungsgemäßen Verfahrens ist dabei die Einstellung bzw. Verbesserung oder Optimierung der Gebrauchseigenschaften. Das praktische Vorgehen in einer möglichen Ausführungsform des Verfahrens wird im Folgenden näher erläutert:
Ausgehend von einem bekannten Dentalkomposit, z.B. VENUS Diamond® (Fa. Heraeus Kulzer) mit dem Hauptvernetzer TCD-di-HEA¹, wird zunächst das Füllstoffsystem modifiziert. Die dort vorhandene weite Partikelgrößenverteilung, vorteilhaft für eine hohe Füllkörperpackungsdichte und hervorragende mechanische Eigenschaften, wird in eine enge Partikelgrößenverteilung um den Mittelwert im Bereich von 1 bis 2 µm umgestellt. Die geringere Packungsdichte und reduzierte Füllstoffoberfläche soll eine bessere Fließfähigkeit (geringere Strukturviskosität) ermöglichen. Der dadurch bedingte höhere Schrumpf des Komposites wird durch Zugabe eines deutlich größeren Prä-Polymerfüllers (mittlere Partikelgröße ca. 30 µm) verbessert. Überraschenderweise haben kleinere Anteile des Präpolymer-Füllers keine nachteiligen Auswirkungen auf die mechanischen Eigenschaften, senken aber die Klebrigkeit sehr deutlich.

Dass kleine polare Moleküle, wie die mit der Formel I zusammengefassten Verbindungen, wie z.B. Wasser, Polyethylenglykol (PEG) mit MG < 250 und Glycerin, einen Einfluss auf die rheologischen Eigenschaften von Kompositen ausüben, ist an sich bekannt. Deren konkreter Einfluss auf bzw. deren gezielter Einsatz in unpolymerisierte(n) Kompositen wurde jedoch bis heute weder systematisch untersucht noch genutzt.

Ausgehend vom Fachwissen sollte im Allgemeinen eine zunehmende Polarität im System zu einer Zunahme der Thixotropie führen. Insbesondere die Silikatoberflächen der marktüblichen Dentalgläser sind hinsichtlich ihrer Chemie bekannt und bilden unter Wasseraufnahme weitere Hydroxygruppen aus, die entsprechend dem Fachwissen zu einer Thixotropie führen. Dieser Effekt ist auch in Dentalkompositen erkennbar, insbesondere bei einer geringen Wasseraufnahme des trockenen Glasmaterials. Obwohl die eingesetzten Dentalglaspartikel zur Verwendung silanisiert werden, bleibt ein Effekt der Thixotropie deutlich erkennbar. Nach einer ersten Wasseraufnahme (Adsorption an der Glaspartikeloberfläche) kehrt sich dieser Effekt jedoch überraschend um und führt zu einer deutlichen Absenkung der Strukturviskosität. Dabei kommt es in den untersuchten Fällen bis ca. 3 bis 4 % Wasserzugabe nicht zur erwarteten Phasenbildung. Die Polymerisation der Komposite wird durch den Anteil Wasser nicht beeinträchtigt.

Ein bevorzugtes Dentalkomposit im Sinne der Erfindung enthält folgende Komponenten: 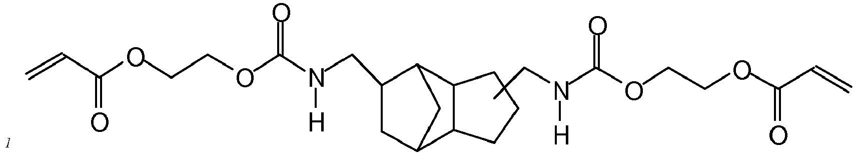
65 bis 75 Gew.-% Glasfüller, vorzugsweise BaAlSilikatglas
1 bis 5 Gew.-% Splitterpolymer
0,1 bis 5 Gew.-% Additiv der Formel H-Y-Z
15 bis 25 Gew.-% Monomermischung, vorzugsweise Urethan(meth)acrylate
15 bis 20 Gew.-% Vernetzer, bevorzugt aus der Gruppe TCD-DI-HEA und HEMA-TMDI.

Derartige Komposite werden bevorzugt nach dem erfindungsgemäßen Verfahren hergestellt oder optimiert, oder sie werden durch Verbesserung bestehender Rezepturen erhalten.

Die Gebrauchseigenschaften eines Komposites sind ein komplexes Zusammenwirken von Thixotropie, Strukturviskosität und Klebrigkeit. Diese Eigenschaften sind ebenfalls ein wichtiger Erfolgsfaktor für die Restaurationstherapie und können mit der erfindungsgemäßen Methode passend eingestellt werden.

Um die Effekte sicher zu beurteilen wurden Prüfmethoden entwickelt, um die Kompositeigenschaften (Fließverhalten/Strukturviskosität und Klebrigkeit) messbar zu erfassen.

Die Texturierung wird z.B. durch eine Fließfähigkeitsmessung bestimmt. Vor der Messung wird eine standardisierte Scherung vorgenommen, so dass der Zustand des Komposites während der Verarbeitung und nach Extrusion aus dem Packmittel simuliert wird. Dann wird die Konsistenz des Komposites - beispielsweise mittels Penetration durch eine definierte Nadel (Sonde) bei einer definierten Penetrationsgeschwindigkeit anhand der Widerstandskraft (entsprechend einer Gewichtskraft) - bestimmt (die Konsistenz kann zwar im Ruhezustand ohne vorbereitende Scherung bestimmt werden; dies ist aber für die praktische Verarbeitung kaum relevant). Es ergibt sich ein Zahlenwert IG (entsprechend einer Gewichtskraft), der in den Tabellen und Diagrammen der Beispiele zu finden ist.

Die Klebrigkeit (Initialklebrigkeit) wird durch eine Messung der Abzugskraft von der Kompositoberfläche bestimmt. Dabei wird auf das vorbereitete Material ein Metall-Prüfstempel definiert aufgesetzt und nach kurzer Verweilzeit definiert abgezogen, wobei die Anhaftung als Kraft gemessen wird. Es ergibt sich ein Zahlenwert IK (entsprechend einer Gewichtskraft), der in den Tabellen und Diagrammen der Beispiele zu finden ist. Er wird in den Diagrammen gegen den Wert IG aufgetragen.

Bei der Entwicklung des erfindungsgemäßen Verfahrens kam noch eine empirisch ermittelte Qualitätsbeurteilung hinzu: Die entsprechenden Kompositmaterialien wurden einem Praxistest unterzogen und deren gute, mittelmäßige oder weniger gute Gebrauchseigenschaften von den Anwendern (Zahntechnikern und Zahnärzten) beurteilt. Die als besonders geeignet bzw. akzeptabel empfundenen Mischungen sind in den Tabellen der Beispiele mit **!** bzw. * gekennzeichnet. Als weniger oder völlig ungeeignet empfundene Mischungen sind mit einem ● für weniger geeignet und einem **Ø** für völlig ungeeignet gekennzeichnet.

Demgemäß wurde festgestellt, dass diejenigen Zusammensetzungen, die sich in den Gebrauchseigenschaften als vorteilhaft herausstellten, IG/IK Quotienten von 2-5 aufweisen, besonders vorteilhafte von 2-3. Diese empirisch ermittelte Grundlage erlaubt es, sowohl die Art der Additive zu optimieren als auch deren mengenmäßigen Anteil an der Zusammensetzung, und außerdem das optimale Mengenverhältnis zwischen Glasfüller und Splitterpolymer, zu ermitteln (vgl. Beispiel 7).

Im Umkehrschluss erlaubt dies eine Prognose der Gebrauchseigenschaften nach Bestimmung der IK und IG Werte. Dem Fachmann wird ein Mittel in die Hand gegeben, die Gebrauchseigenschaften gezielt zu beeinflussen, und er ist dabei nicht auf Versuch und Irrtum angewiesen. Der Umfang der Praxistests kann bei Anwendung des erfindungsgemäßen Verfahrens weitgehend reduziert werden.

Im Vorliegenden wird unter "fließfähiges Dentalkomposit" bevorzugt ein verformbares Material, nicht unbedingt aber ein dünnflüssiges Dentalmaterial, verstanden. Insbesondere wird der Grad der "Fließfähigkeit" geeigneter Materialien im Allgemeinen dadurch bestimmt, dass Messmethoden für Festigkeit/Konsistenz und Klebrigkeit anwendbar sind.

### Initialklebrigkeit

Gemessen wird die Klebrigkeit (Klebekraft) in Gramm, die beim Abzug eines Messstempels, welcher mit einer definierten Kraft auf die Probe gedrückt wird, benötigt wird.

### Festigkeit

Gemessen wird die einer festgelegten Eindringtiefe eines Messstempels entgegengesetzte Kraft in Gramm.

Die folgenden Beispiele erläutern die Erfindung näher. Teile- und Prozentangaben beziehen sich, sofern nicht anders angegeben, auf das Gewicht.

### Beispiele und Beschreibung der Figuren

Es werden zahlreiche Kompositvarianten hergestellt. Eine davon (ohne Splitterpolymer) setzt sich wie folgt zusammen: 1% TEGDMA, 6% HEMA-TMDI, 15% TCD-di-HEA, 6% SiO₂, 72% Dentalglas. Daraus sind die in den Zusammensetzungen der Tabellen nicht explizit aufgeführten Komponenten zu ersehen.

Die aus den Tabellen zu ersehenden Kompositvarianten werden erfindungsgemäß auf die Klebrigkeit und das Fließverhalten geprüft. Letzteres wird über den Grad der Festigkeit ermittelt. Das Vorgehen ist wie folgt:
Proben: Mit einem Zentrifugalmischer frisch homogenisierte Proben der zu messenden Pastenmischung.
Umgebungsbedingungen: Messraum mit definiertem Klima.

### Beispiel A: Prüfung der Initialklebrigkeit hochviskoser Pasten

Gemessen wird die Klebrigkeit (Klebekraft) in Gramm, die beim Abzug eines Messstempels, welcher mit einer definierten Kraft auf die Probe gedrückt wird, benötigt wird.

### Gerät: Kraft-Weg Messgerät mit definiertem Prüfstempel

Auf die mit einem Zentrifugalmischer frisch homogenisierte Probe wird der Messstempel definiert aufgesetzt und die Abzugskraft ermittelt. Als Ergebnis wird der Mittelwert der Klebekraft in Pond bzw. Gramm [g] angegeben.

### Beispiel B: Prüfung der Festigkeit hochviskoser Pasten

Gemessen wird die einer festgelegten Eindringtiefe eines Messstempels entgegengesetzte Kraft in Gramm.

### Gerät: Kraft-Weg Messgerät mit definiertem Prüfstempel.

Als Ergebnis wird der Mittelwert der Kraft in Pond bzw. Gramm [g] angegeben.

### Bespiele 1, 2 und 3: Einbezug der im Praxistest ermittelten Beurteilungen

Die wie oben beschrieben ermittelten Resultate der IG und IK Werte sowie die Ergebnisse der Beurteilung durch Anwender in Praxistests (Symbole !, *, ●, Ø für optimiert, akzeptabel, weniger geeignet und ungeeignet) sind am Beispiel von Variationen der Rezeptur des Dentalkomposits VENUS Diamond® (Heraeus Kulzer GmbH; Geschäftsbereich Dentalprodukte - Heraeus Dental) in Beispiel 1, Tabelle 1, zusammengestellt.

Die rechnerisch ermittelten Werte IG/IK wurden mit den Symbol - für weniger geeignet, * für akzeptabel) und **!** für freigegeben (bzw. als optimiert zu betrachten) gekennzeichnet.

Die einzelnen Tabellen des Beispiels 2 zeigen Zusammensetzungen mit verschiedenen Glas- und Splitter-Anteilen und einem ansteigenden Wassergehalt, wobei das Wasser in definierten Mengen als Additiv zugesetzt wurde. Die Bewertungsresultate in Bezug auf die Gebrauchseigenschaften sind wieder mit den Symbolen !, *, ● und Ø gekennzeichnet.

### Beispiel 3 zeigt analog Beispiel 1 den Einfluss der Additive PEG und MEMO.

Die Beziehungen zwischen den ermittelten Werten für Klebrigkeit und Texturierung zeigen anschaulich das wissenschaftliche Herangehen an die Optimierung von Dentalkompositen. Sie sind in den Diagrammen der Figuren zusammengefasst:
Fig. 1 (Beispiel 4) zeigt das Verhalten von Konsistenz und Klebrigkeit bei Zugabe von Glasfüllstoff und konstantem Anteil Präpolymer-Füllstoff ohne Additiv.
Fig. 2 (Beispiel 5) zeigt unter den Bedingungen des Beispiels 4 den Effekt von Wasser als Additiv.
Fig. 3 (Beispiel 6) zeigt die Effekte der Variation des Präpolymer-Füllstoffanteils und des Wasseranteils bei konstantem Glasfüllstoff-Anteil.
Fig. 4 (Beispiel 7) zeigt, wie sich die Optima aus den Beispielen 5 und 6 via Auftragung der IG/IK Werte ermitteln lassen.
Fig.5 (Beispiel 8 zeigt das Verhalten von Konsistenz und Klebrigkeit bei Zugabe von Glasfüllstoff und konstantem Anteil Präpolymer-Füllstoff ohne und mit 3 Mengenvarianten des Additivs MEMO.
Fig. 6 (Beispiel 9) zeigt die Auswertung des Beispiels 8 via Auftragung der IG/IK Werte.

Abkürzungen:
**i)** F% = Füllstoffanteil in % (Masseanteil aller Füllstoffe: Dentalglas und Prä-Polymer)
**ii)** Stabw = Standardabweichung
**iii)** GRM = Grundmasse (Komposit ohne farbgebende Pigmentierung)
**iv)** o. = ohne
**v)** Std. = Standard
**vi)** Splitter = Prä-Polymer Füllstoff
**vii)** HEMA= 2-Hydroxyethylmethacrylat
**viii)** TMDI = 2,2,4(2,4,4)-Trimethyl-1,6-hexanediisocyanat
**ix)** HEMA/TMDI = Additionsprodukt aus **vii** und **viii**
**x)** TEGDMA =Triethylenglykoldimethacrylat
**xi)** TCD-di-HEA = (Bis-(acryloyloxymethyl)tricyclo[5.2.1.02,6]decan)

### Beispiel 1: Einfluss durch Additiv Wasser - Messwerte / Beurteilungen

| | | | | | | **Messergebnisse Texturen Analysen** | | | | | | |
|---|---|---|---|---|---|---|---|---|---|---|---|---|
| **Materialbezeichnung** | **Bemerkung** | **Wasser [%]** | **Präpolymer [%]** | **Glas [%]** | **F%** | **IG** | | **IK** | | **Bemerkungen** | **IG** | Koeffizient IG/IK |
| | | | | | | Kraft, g | Stab w | Kraft ,g | Stab w | Akzeptanzbereich | 110-170 | 1,25-5,6 |
| **SOCO** | | | | | | **120-170** | | **30-100** | | Freigabebereich | 125-160 | 1,7-4,16 |
| **GRM** | | | | | | | | | | | | |
| SOCO **GRM** VP 050711 KR1 | **o. H₂O - Std**. **Paste** | **0** | **2** | **66** | **68** | **64●** | 3 | **187Ø** | 6 | | 64 | 0,3- |
| SOCO **GRM** VP 050711 KR1/ K1 | | **0** | **2** | **67** | **69** | **88●** | 4 | **181Ø** | 7 | | 88 | 0,5- |
| SOCO **GRM** VP 050711 KR1/ K2 | | **0** | **2** | **68** | **70** | **102●** | 2 | **144Ø** | 7 | | 102 | 0,7- |
| SOCO **GRM** VP 050711 KR1/ K3 | | **0** | **2** | **69** | **71** | **181 Ø** | 7 | **81!** | 7 | | 181 | 2,2! |
| SOCO **GRM** VP 050711 KR1/ K4 | | **0** | **2** | **70** | **72** | **307 Ø** | 6 | **35!** | 4 | | 307 | 8,8- |
| | | | | | | | | | | | | |
| SOCO **GRM** VP 140611 KR2 | **o. H₂O / G 018-053** | **0** | **0** | **68** | **68** | **64●** | 2 | **200Ø** | 6 | | 64 | 0,3- |
| SOCO **GRM** VP 140611 KR2/ K1 | | **0** | **0** | **69** | **69** | **90●** | 4 | **180Ø** | 12 | | 90 | 0,5- |
| SOCO **GRM** VP 140611 KR2/ K2 | | **0** | **0** | **70** | **70** | **118●** | 4 | **139Ø** | 9 | | 118 | 0,8- |
| SOCO **GRM** VP 140611 KR2/ K3 | | **0** | **0** | **71** | **71** | **200 Ø** | 3 | **71!** | 6 | | 200 | 2,8! |
| SOCO **GRM** VP 140611 KR2/ K4 | | **0** | **0** | **72** | **72** | **3360** | 13 | **35!** | 3 | | 336 | 9,6- |
| | | | | | | | | | | | | |
| SOCO **GRM** VP 140611 KR2 | **o. H₂O / Splitter** | **0** | **2** | **66** | **68** | **64●** | 2 | **200Ø** | 6 | | 64 | 0,3- |
| SOCO **GRM** VP 140611 KR2/SP/K1 | | **0** | **3** | **66** | **69** | **87●** | 3 | **178Ø** | 6 | | 87 | 0,5- |
| SOCO **GRM** VP 140611 KR2/SP/K2 | | **0** | **4** | **66** | **70** | **110●** | 1 | **150Ø** | 8 | | 110 | 0,7- |
| SOCO **GRM** VP 140611 KR2/SP/K3 | | **0** | **5** | **66** | **71** | **142!** | 5 | **103Ø** | 5 | | 142 | 1,4* |
| SOCO **GRM** VP 140611 KR2/SP/K4 | | **0** | **6** | **66** | **72** | **195 Ø** | 6 | **74!** | 3 | | 195 | 2,6! |
| | | | | | | | | | | | | |
| SOCO **GRM** VP 140611 KR3 | **0,5% H₂O/G018-053 UF** | **0,5** | **2** | **66** | **68** | **44●** | 1 | **17 Ø** | 10 | | 44 | 0,3- |
| SOCO **GRM** VP 140611 KR3/ K1 | | **0,5** | **2** | **67** | **69** | **59●** | 2 | **162Ø** | 11 | | 59 | 0,4- |
| SOCO **GRM** VP 140611 KR3/ K2 | | **0,5** | **2** | **68** | **70** | **110●** | 3 | **112Ø** | 12 | | 110 | 1,0- |
| SOCO **GRM** VP 140611 KR3/ K3 | | **0,5** | **2** | **69** | **71** | **164!** | 8 | **61!** | 5 | | 164 | 2,7! |
| SOCO **GRM** VP 140611 KR3/ K4 | | **0,5** | **2** | **70** | **72** | **242Ø** | 7 | **29** | 3 | | 242 | 8,3- |
| | | | | | | | | | | | | |
| SOCO **GRM** VP 140611 KR3 | **0,5% H₂O / Splitter** | **0,5** | **2** | **66** | **68** | **44●** | 1 | **175Ø** | 10 | | 44 | 0,3- |
| SOCO **GRM** VP 200611 KR1 | **0,5% H₂O / Splitter** | **0,5** | **2** | **66** | **68** | **43●** | 2 | **166Ø** | | 10 | 43 | 0,3- |
| SOCO **GRM** VP 200611 KR1/SP/ K1 | | **0,5** | **3** | **66** | **69** | **58●** | 3 | **158Ø** | 8 | | 58 | 0,4- |
| SOCO **GRM** VP 200611 KR1/SP/ K2 | | **0,5** | **4** | **66** | **70** | **76●** | 3 | **125Ø** | 5 | | 76 | 0,6- |
| SOCO **GRM** VP 200611 KR1/SP/ K3 | | **0,5** | **5** | **66** | **71** | **101●** | 3 | **78!** | 3 | | 101 | 1,3* |
| SOCO **GRM** VP 200611 KR1/SP/ K4 | | **0,5** | **6** | **66** | **72** | **133!** | 3 | **60!** | 2 | | 133 | 2,2! |
| | | | | | | | | | | | | |
| SOCO **GRM** VP 270611 KR | **1,0% H₂O / G018-053 UF** | **1** | **2** | **66** | **68** | **50●** | 1 | **104Ø** | 9 | | 50 | 0,5- |
| SOCO **GRM** VP 270611 KR/ K1 | | **1** | **2** | **67** | **69** | **58●** | 2 | **87!** | 8 | | 58 | 0,7- |
| SOCO **GRM** VP 270611 KR/ K2 | | **1** | **2** | **68** | **70** | **68●** | 2 | **65!** | 4 | | 68 | 1,0- |
| SOCO **GRM** VP 270611 KR/ K3 | | **1** | **2** | **69** | **71** | **119●** | 4 | **37!** | 1 | | 119 | 3,2! |
| SOCO **GRM** VP 270611 KR/ K4 | | **1** | **2** | **70** | **72** | **162!** | 5 | **22** | 7 | | 162 | 7,4- |
| | | | | | | | | | | | | |
| SOCO **GRM** VP 220611 KR. | **1,0% H₂O /-Splitter** | **1** | **2** | **66** | **68** | **51●** | 2 | **100!** | 12 | | 51 | 0,5- |
| SOCO **GRM** VP 220611 KR/SP/ K1 | | **1** | **3** | **66** | **69** | **60●** | 2 | **73!** | 5 | | 60 | 0,8- |
| SOCO **GRM** VP 220611 KR/SP/ K2 | | **1** | **4** | **66** | **70** | **75●** | 2 | **58!** | 5 | | 75 | 1,3* |
| SOCO **GRM** VP 220611 KR/SP/ K3 | | **1** | **5** | **66** | **71** | **103●** | 2 | **45!** | 5 | | 103 | 2,3! |

| **Materialbezeichnung** | **Bemerkung** | **Wasser [%]** | **Präpolymer [%]** | **Glas [%]** | **F%** | **IG** | | **IK** | | **Bemerkungen** | **IG** | Koeffizient IG/IK |
|---|---|---|---|---|---|---|---|---|---|---|---|---|
| SOCO **GRM** VP 220611 KR/SP/ K4 | | **1** | **6** | **66** | **72** | **145!** | 4 | **26** | 4 | | 145 | 5,6* |
| SOCO **GRM** VP300611 KR | **1,5% H₂O/G018-053 UF** | **1,5** | **2** | **66** | **68** | **56●** | 3 | **93!** | 10 | | 56 | 0,6- |
| SOCO **GRM** VP300611 KR/ K1 | | **1,5** | **2** | **67** | **69** | **77●** | 2 | **46!** | 4 | | 77 | 1,7* |
| SOCO **GRM** VP300611 KR/ K2 | | **1,5** | **2** | **68** | **70** | **118●** | 1 | **24** | 4 | | 118 | 4,9* |
| SOCO **GRM** VP300611 KR/ K3 | | **1,5** | **2** | **69** | **71** | **177 Ø** | 3 | **12** | 1 | | 177 | 14,8- |
| SOCO **GRM** VP300611 KR/ K4 | | **1,5** | **2** | **70** | **72** | **297 Ø** | 9 | **4** | 1 | | 297 | 74,3**-** |
| | | | | | | | | | | | | |
| SOCO **GRM** VP 040711 KR1 | **1,5% H₂O / Splitter** | **1,5** | **2** | **66** | **68** | **64●** | 5 | **81!** | 9 | | 64 | 0,8- |
| SOCO **GRM** VP 040711 KR1 /SP/ K1 | | **1,5** | **3** | **66** | **69** | **72●** | 3 | **55!** | 3 | | 72 | 1,3* |
| SOCO **GRM** VP 040711 KR1 /SP/ K2 | | **1,5** | **4** | **66** | **70** | **99●** | 3 | **38!** | 3 | | 99 | 2,6! |
| SOCO **GRM** VP 040711 KR1 /SP/ K3 | | **1,5** | **5** | **66** | **71** | **117●** | 2 | **24** | 3 | | 117 | 4,9* |
| SOCO **GRM** VP 040711 KR1 /SP/ K4 | | **1,5** | **6** | **66** | **72** | **157!** | 3 | **11** | 1 | | 157 | 14,3- |
| | | | | | | | | | | | | |
| SOCO **GRM** VP 270611 KR/ K1 | **1,0% H₂O/ Splitter** | **1** | **2** | **67** | **69** | **58●** | 2 | **87!** | 8 | | 58 | 0,7- |
| SOCO **GRM** VP 160811 Rei1 FK67/3 1% H2O | | **1** | **3** | **67** | **70** | **92●** | 3 | **38!** | 3 | | 92 | 2,4! |
| SOCO **GRM** VP 160811 Rei2 FK67/4 1% H2O | | **1** | **4** | **67** | **71** | **135** | 3 | **27** | 1 | | 135 | 5,0***** |
| SOCO **GRM** VP 170811 Rei1 FK67/5 1% H2O | | **1** | **5** | **67** | **72** | **187Ø** | 2 | **7** | 2 | | 187 | 26,7**-** |
| SOCO **GRM** VP 170811 Rei2 FK67/6 1% H2O | | **1** | **6** | **67** | **73** | **265Ø** | 9 | **3** | 1 | | 265 | 88,3- |
| | | | | | | | | | | | | |
| SOCO **GRM** VP 270611 KR/ K2 | **1,0% H₂O/Split-ter** | **1** | **2** | **68** | **70** | **68●** | 2 | **65!** | 4 | | 68 | 1,0- |
| SOCO **GRM** VP 170811 Rei2 FK68/3 1% H2O | | **1** | **3** | **68** | **71** | **178Ø** | 4 | **14** | 3 | | 178 | 12,7- |
| SOCO **GRM** VP 180811 Rei2 FK68/4 1% H2O | | **1** | **4** | **68** | **72** | **208Ø** | 5 | **11** | 2 | | 208 | 18,9- |
| | | | | | | | | | | | | |
| SOCO GRM VP 140611 KR3/ K1 | **0,5% H₂O/ Splitter** | **0,5** | **2** | **67** | **69** | **59●** | 2 | **162Ø** | 11 | | 59 | 0,4- |
| SOCO **GRM** VP 180811 Rei2 FK63/3 0,5% H2O | | **0,5** | **3** | **67** | **70** | **92●** | 2 | **115Ø** | 9 | | | |
| SOCO **GRM** VP 180811 Rei3 FK67/4 0,5% H2O | | **0,5** | **4** | **67** | **71** | **130!** | 4 | **60!** | 3 | | 130 | 2,2! |
| SOCO **GRM** VP 190811 Rei1 FK67/5 0,5% H2O | | **0,5** | **5** | **67** | **72** | **167!** | 3 | **27** | 3 | | 167 | 6,2- |
| | | | | | | | | | | | | |
| SOCO **GRM** VP 140611 KR3/ K2 | **0,5% H₂O/Sp litter** | **0,5** | **2** | **68** | **70** | **110●** | 3 | **112Ø** | 12 | | 110 | 1,0- |
| SOCO **GRM** VP 190811 Rei2 FK68/3 0,5,% H2O | | **0,5** | **3** | **68** | **71** | **132!** | 2 | **57!** | 1 | | 132 | 2,3! |
| SOCO **GRM** VP 190811 Rei3 FK68/4 0,5% H2O | | **0,5** | **4** | **68** | **72** | **194Ø** | 1 | **28** | 1 | | 194 | 6,9- |

### Beispiel 2: Einfluss durch Additiv Wasser - Matrixauswertung

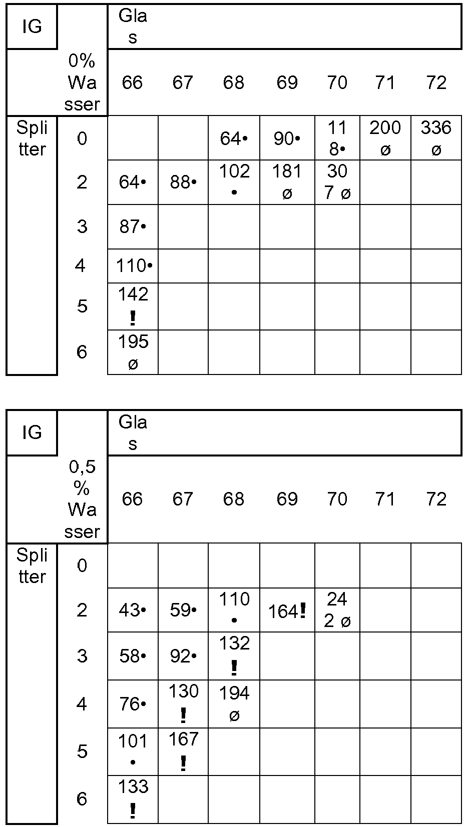

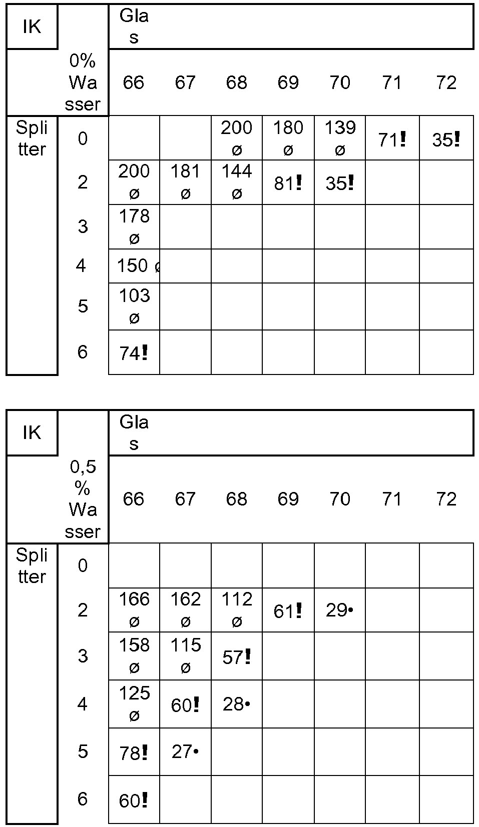

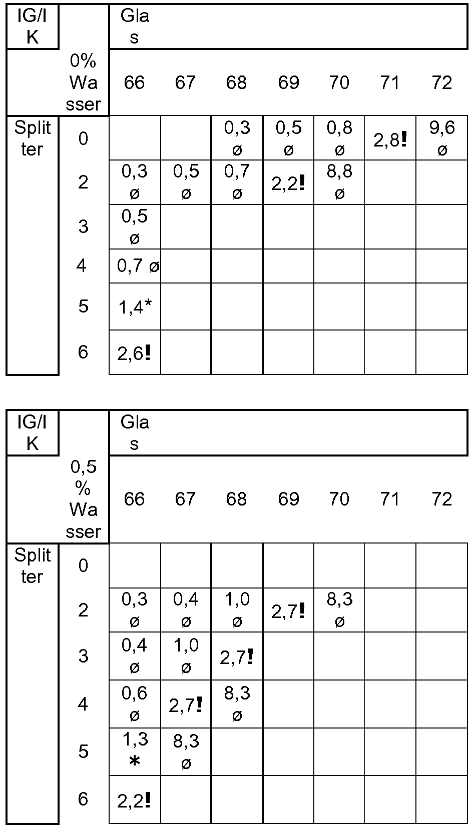

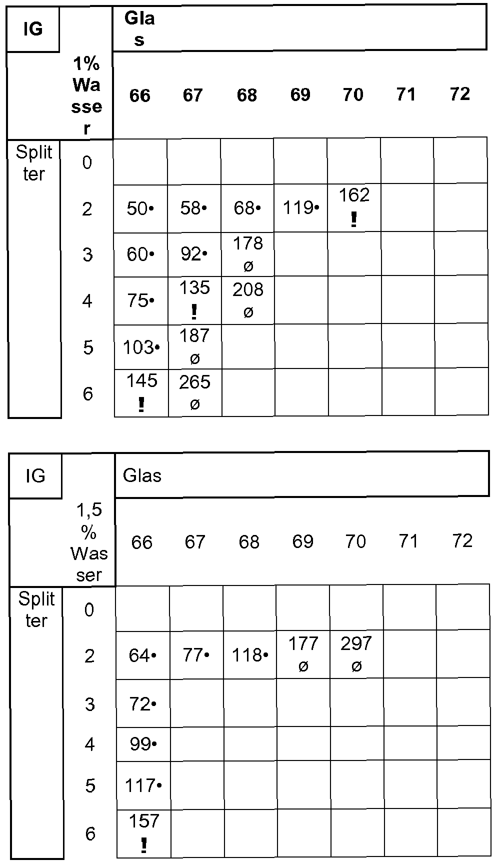

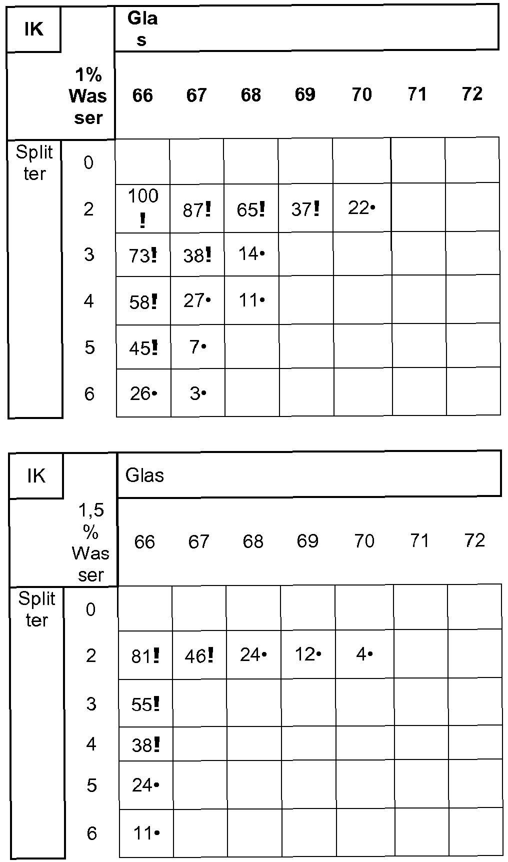

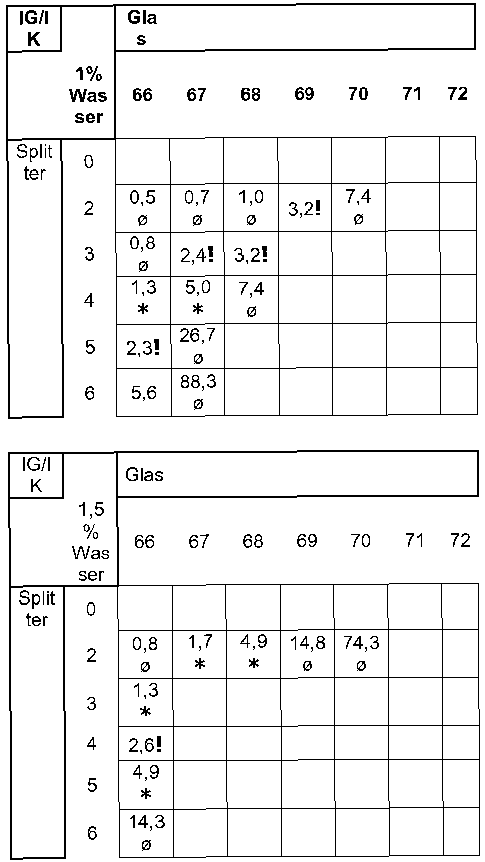

### Beispiel 3: Einfluss durch hydroxyfunktionelle Additive (PEG200, Glycerin, MEMO) - Messwerte und Beurteilungen

| | | **Messergebnisse Texturen Analysen** | | | | | | | | | |
|---|---|---|---|---|---|---|---|---|---|---|---|
| **Material** | | **Präpolymer [%]** | **Glas [%]** | **F%** | **IG** | | **IK** | | **Bemerkungen** | **IG** | Koeffizient IG/IK |
| | | | | | Kraft, g | Stabw | Kraft,g | Stabw | | | |
| **SOCO** | | | | | **130-170** | | **30-100** | | Freigabebereich | 130-170 | 1,3-5,67 |
| **GRM** | | | | | | | | | | | |
| | | | | | | | | | | | |
| SOCO **GRM** VP 250711 Rei | **Glycerin** | | | | | | | | | | |
| SOCO **GRM** VP 100811 Rei1 | **0,5% Glycerin** | **2** | **67** | **69** | **135!** | 3 | **87!** | 5 | | 135! | 1,6* |
| SOCO **GRM** VP 100811 Rei2 | **1,0% Glycerin** | **2** | **67** | **69** | **215 Ø** | 4 | **15●** | 2 | | 215 | 14,3- |
| SOCO **GRM** VP 110811 Rei1 | **1,5% Glycerin** | **2** | **67** | **69** | **268 Ø** | 6 | **15●** | 2 | | 268 | 17,9- |
| | | | | | | | | | | | |
| SOCO **GRM** VP 250711 Rei1 | **0,5% Glycerin** | **2** | **69** | **71** | **321 Ø** | 6 | **8●** | 0,5 | | 321 | 40,1- |
| SOCO **GRM** VP 250711 Rei2 | **1,0% Glycerin** | **2** | **69** | **71** | **414 Ø** | 8 | **6●** | 2 | | 414 | 69,0- |
| SOCO **GRM** VP 250711 Rei3 | **1,5% Glycerin** | **2** | **69** | **71** | **534 Ø** | 38 | **10●** | 1 | | 534 | 53,4- |
| | | | | | | | | | | | |
| SOCO **GRM** VP 280711 Rei | **PEG 200** | | | | | | | | | | |
| SOCO **GRM** VP 110811 Rei2 | **0,5% PEG 200** | **2** | **67** | **69** | **87●** | 2 | **76!** | 6 | | 87 | 1,1- |
| SOCO **GRM** VP 110811 Rei3 | **1,0% PEG 200** | **2** | **67** | **69** | **94●** | 2 | **70!** | 7 | | 94 | 1,3* |
| SOCO **GRM** VP 110811 Rei4 | **1,5% PEG 200** | **2** | **67** | **69** | **72●** | 3 | **72!** | 8 | | 72 | 1,0- |
| | | | | | | | | | | | |
| SOCO **GRM** VP 280711 Rei1 | **0,5% PEG 200** | **2** | **69** | **71** | **222 Ø** | 5 | **30!** | 3 | | 222 | 7,4- |
| SOCO **GRM** VP 280711 Rei2 | **1,0% PEG 200** | **2** | **69** | **71** | **171 Ø** | 7 | **26●** | 3 | | 171 | 6,6- |
| SOCO **GRM** VP 280711 Rei3 | **1,5% PEG 200** | **2** | **69** | **71** | **166!** | 1 | **29●** | 3 | | 166 | 5,7- |
| | | | | | | | | | | | |
| | | | | | | | | | | | |
| SOCO **GRM** VP 250711 Rei | **MEMO Silan GF31** | | | | | | | | | | |
| SOCO **GRM** VP 280711 Rei4 | **0,5% GF31** | **32** | **69** | **71** | **112●** | 2 | **150 Ø** | 5 | | 112 | 0,7- |
| SOCO **GRM** VP 250711 Rei2 | **1,0% GF31** | **Messungen entfallen zu weich** | | | | | | | | | |
| SOCO **GRM** VP 250711 Rei3 | **1,5% GF31** | | | | | | | | | | |
| | | | | | | | | | | | |
| SOCO **GRM** VP 140611 KR2/ K1 | **o. H₂O / G 018-053** | **0** | **69** | **69** | **90●** | 4 | **180 Ø** | 12 | | 90 | 0,5- |
| SOCO **GRM** VP 140611 KR2/ K2 | **o. H₂O / G 018-053** | **0** | **70** | **70** | **118●** | 4 | **139 Ø** | 9 | | 118 | 0,8- |
| SOCO **GRM** VP 140611 KR2/ K3 | **o. H₂O / G 018-053** | **0** | **71** | **71** | **200 Ø** | 3 | **71!** | 6 | | 200 | 2,8! |
| SOCO **GRM** VP 140611 KR2/ K4 | **o. H₂O / G 018-053** | **0** | **72** | **72** | **336 Ø** | 13 | **35!** | 3 | | 336 | 9,6- |
| | | | | | | | | | | | |
| SOCO **GRM** VP 110811 Rei2 | **0,5% PEG 200** | **2** | **68** | **70** | **158!** | 5 | **39!** | 3 | | 158 | 4,1! |
| SOCO **GRM** VP 110811 Rei3 | **1,0% PEG 200** | **2** | **68** | **70** | **143!** | 1 | **36!** | 3 | | 143 | 4,0! |
| SOCO **GRM** VP 110811 Rei4 | **1,5% PEG 200** | **2** | **68** | **70** | **151!** | 3 | **35!** | 2 | | 151 | 4,3* |

### Beispiel 3a: Einfluss durch hydroxyfunktionelle Additive - Matrixauswertung

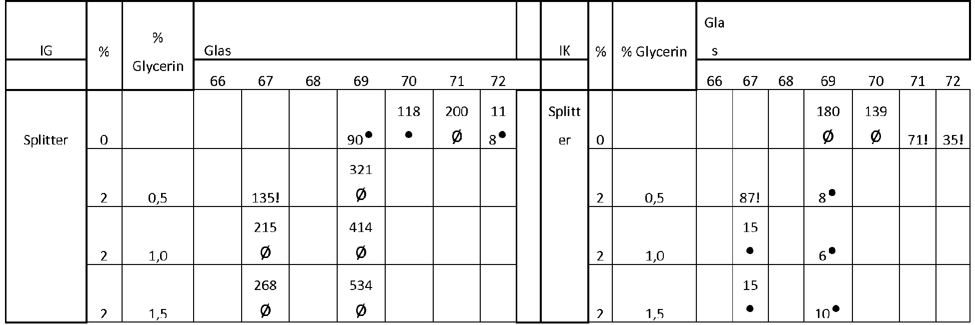

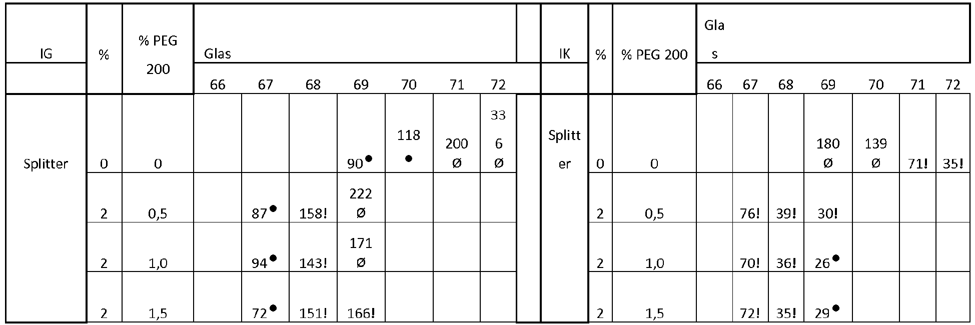

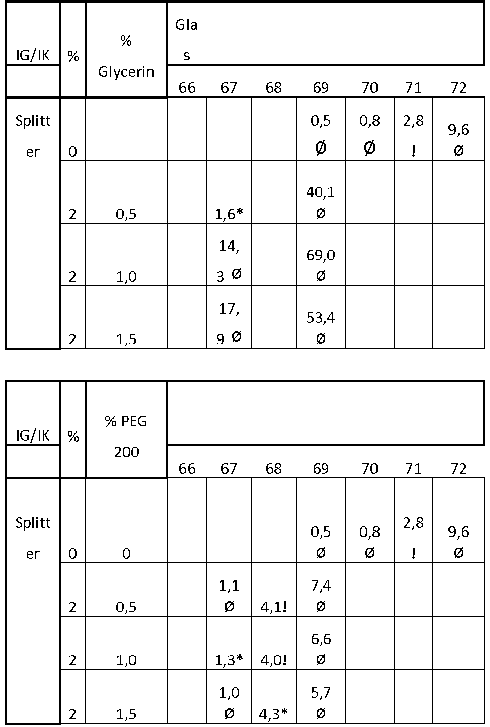

### Fortsetzung Beispiel 3a

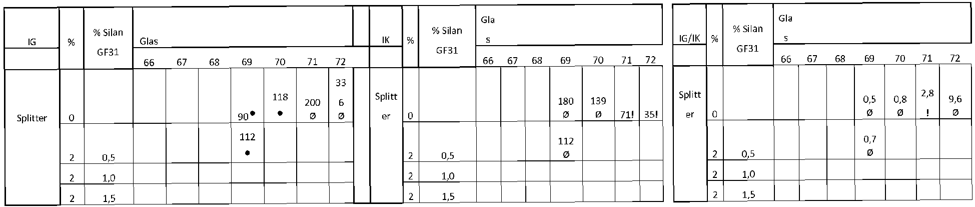

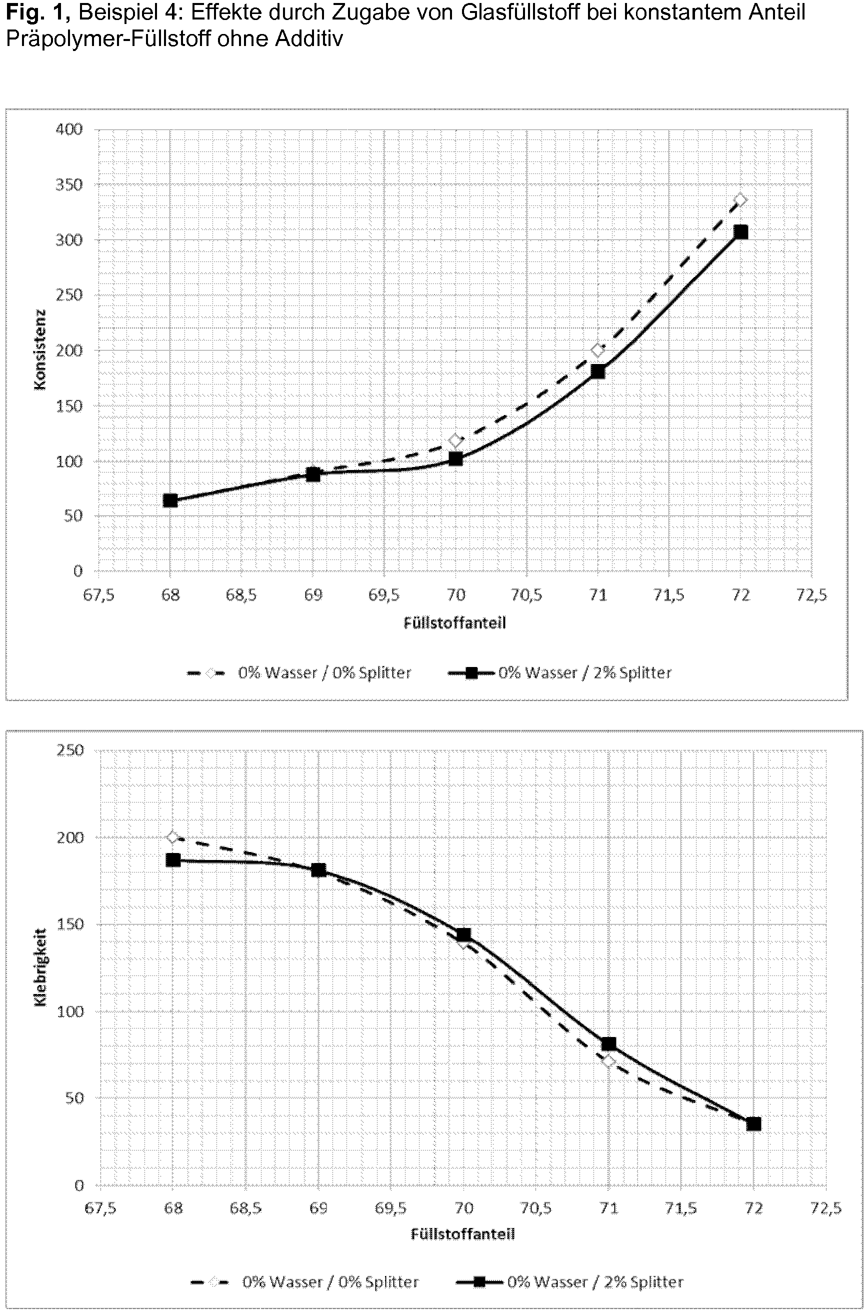

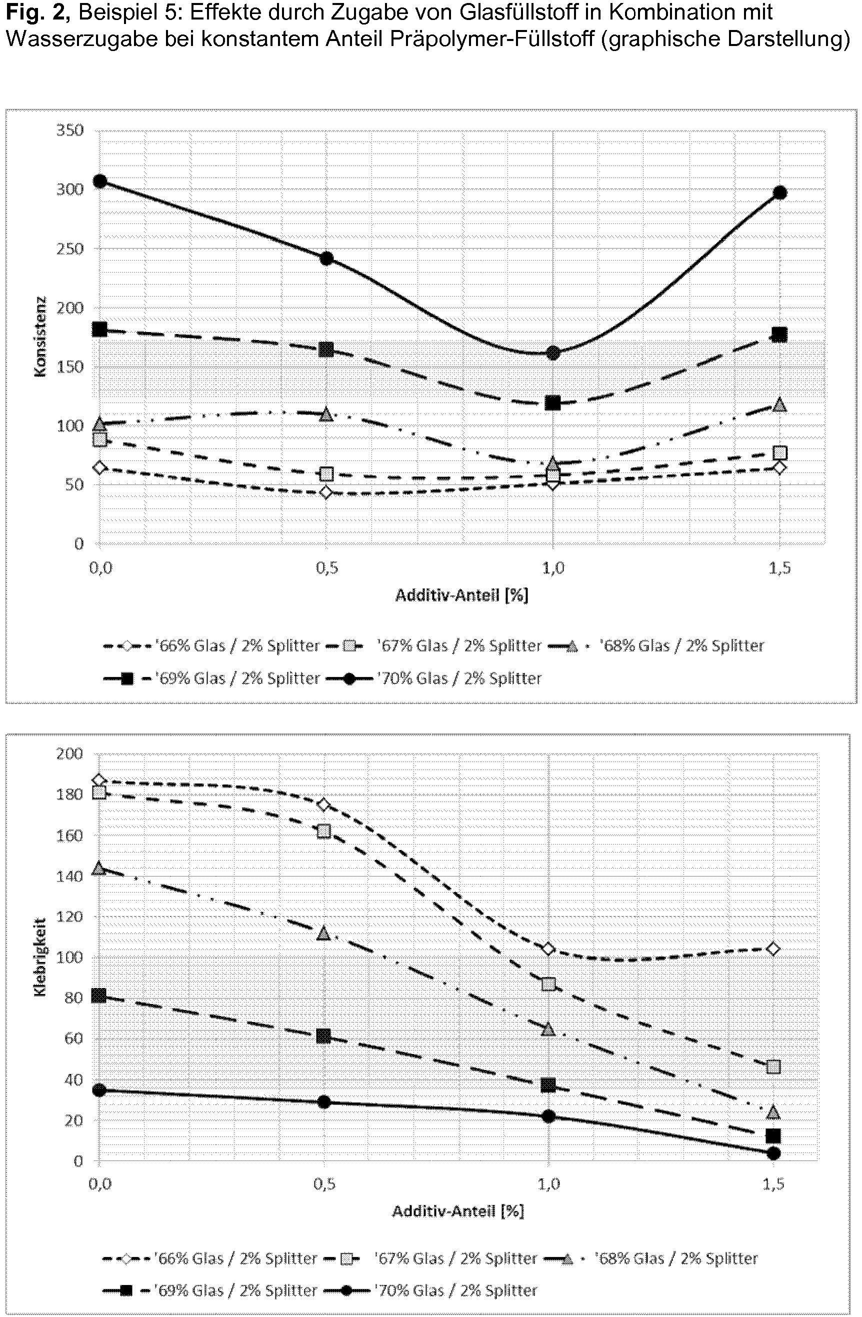

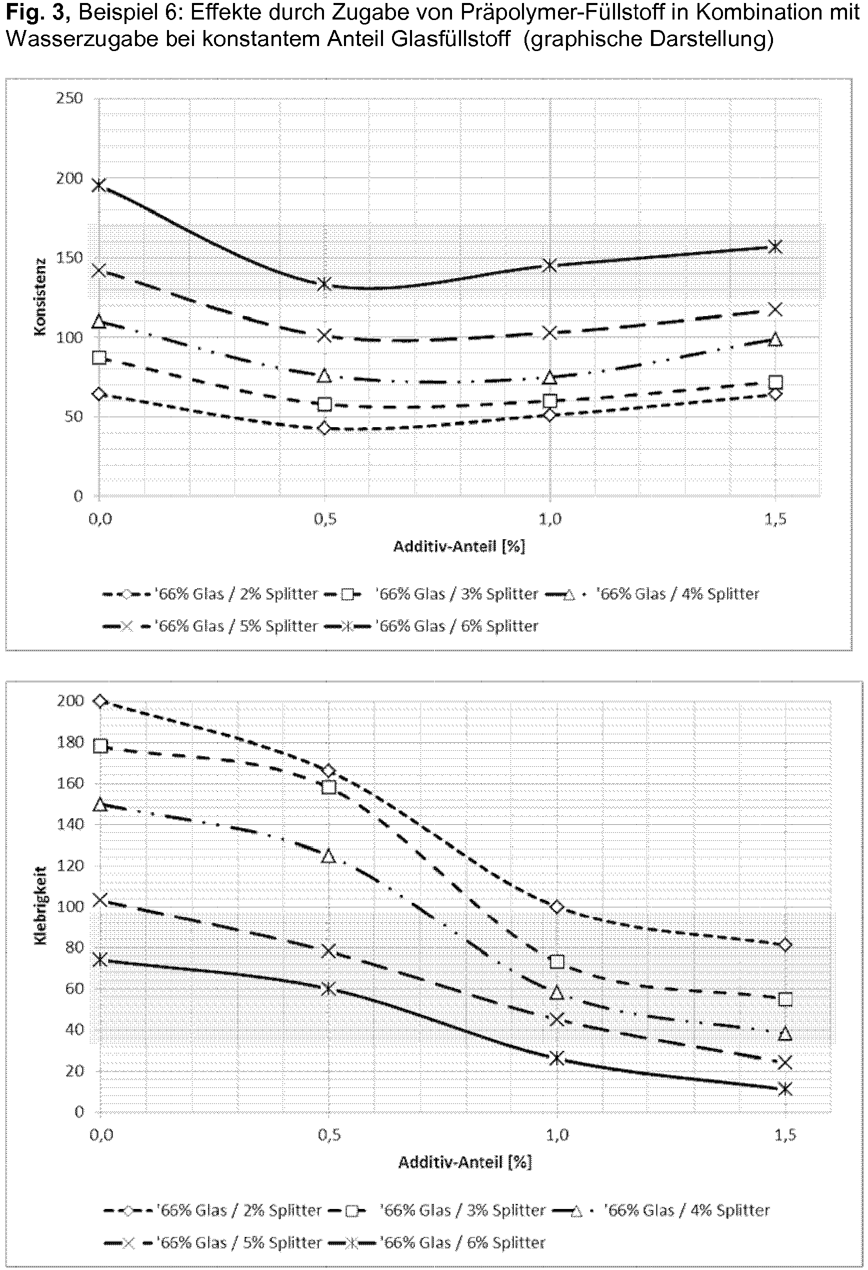

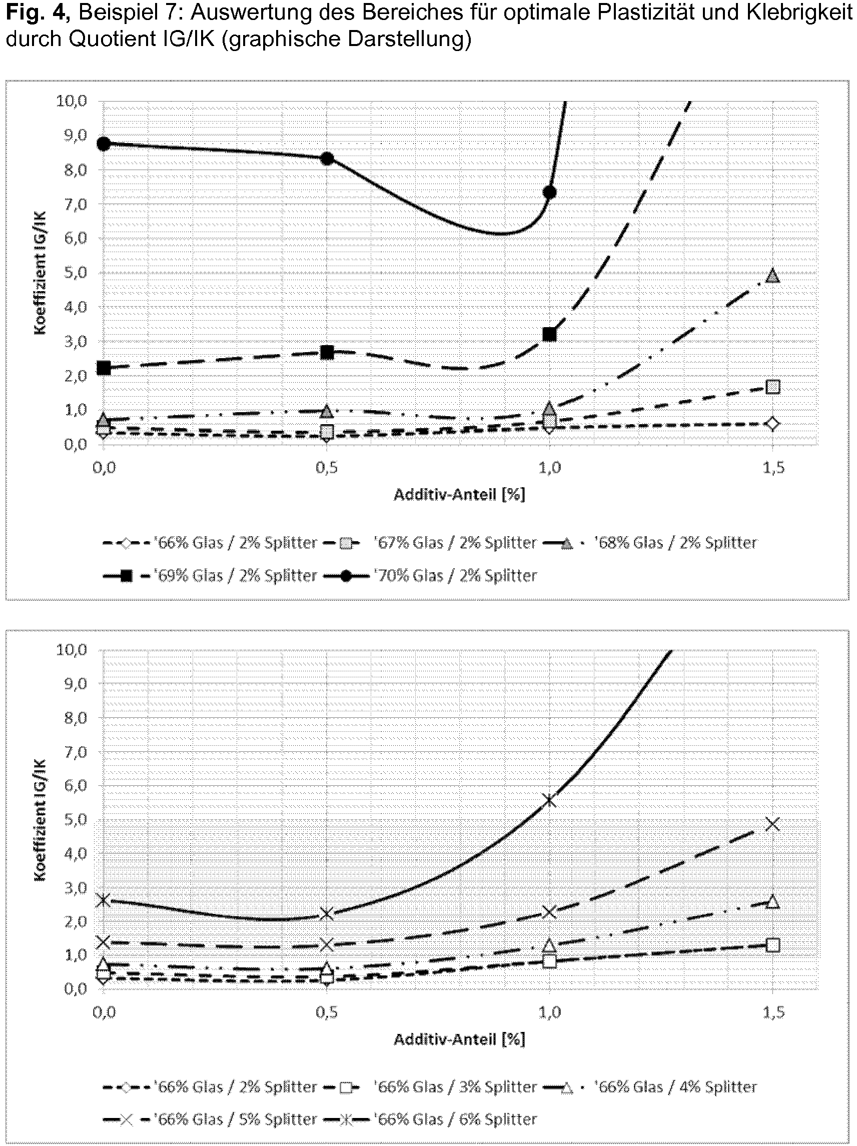

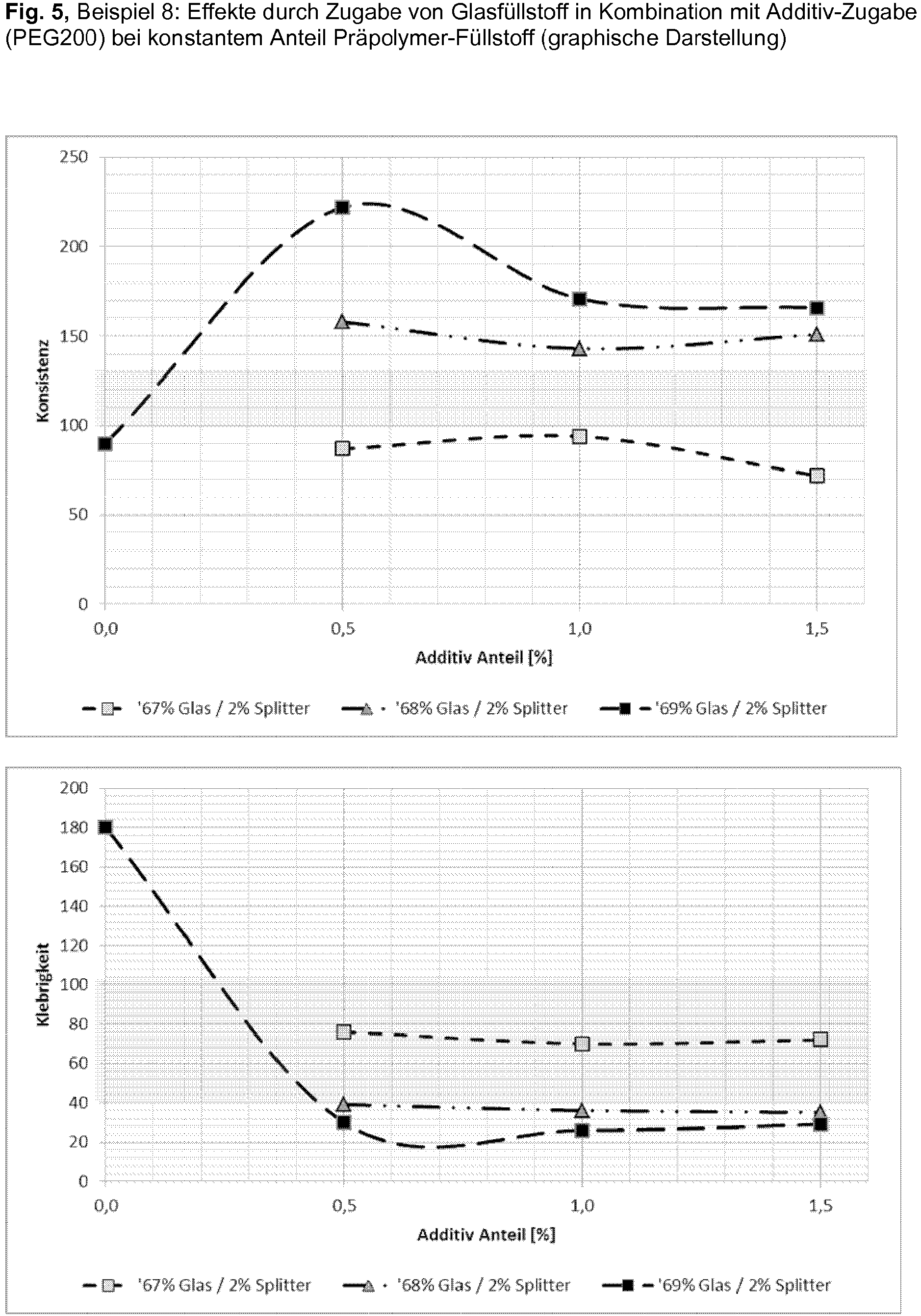

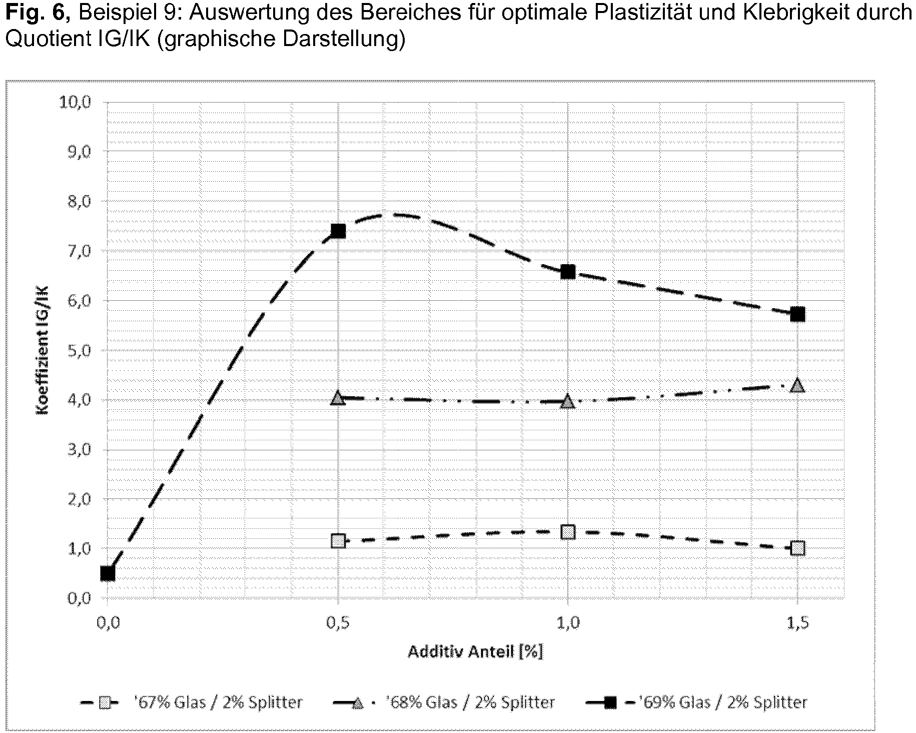

## Patentansprüche

1. Verfahren zur Einstellung oder Verbesserung oder Optimierung der Gebrauchseigenschaften von Füllstoff(e) und Glasfüller(n) enthaltenden, fließfähigen Dentalkompositen, bei dem
i) mindestens ein Splitterpolymerisat-Füller **A1** zugegeben wird,
ii) der Anteil der Komponente **A1** variiert wird,
iii) mindestens ein hydroxyfunktionalisiertes Additiv **A2** mit einem Molekulargewicht < 250 g/mol zugegeben wird, wobei
das Additiv **A2** der Formel H-Y-Z (I) entspricht, worin bedeuten:
Y = -O-, -S-, -CO-, -OSi(OR¹)₂-, -OE
Z = H, OH, SH, NH₂, COOH, COOR²
E = -CₙHₘOₚ-
R¹ = H, C₁-C₄-Alkyl
R² = C₁-C₁₅-Alkyl, durch ein oder mehrere O-Atome unterbrochenes C₂-C₁₄-Alkyl,
n = 2 bis 5
m = 4 bis 10
p = n,
wobei jeweils maximal 5 Gew.-% des mindestens einen Additivs **A1** und **A2,** bezogen auf die Gesamtzusammensetzung des Dentalkomposits, zugegeben werden,
iv) eine Messgröße IK [Kraft, Gramm] für die Klebrigkeit gemessen wird, eine Messgröße IG [Kraft, Gramm] für die Texturierung oder Plastizität oder Konsistenz gemessen wird und beide Messgrößen zueinander in Beziehung gesetzt werden, wobei
- der Wert IG für die Texturierung/Plastizität/Konsistenz aus dem Kraftaufwand bei Durchtritt durch eine definierte Verengung oder
aus der einer festgelegten Eindringtiefe eines Messstempels entgegengesetzten Kraft ermittelt wird, und
- der Wert IK für die Klebrigkeit aus der Abzugskraft beim Abzug des Messstempels von der Kompositoberfläche, welcher mit einer definierten Kraft auf die Probe gedrückt worden ist, ermittelt wird, und sodann
v) ein optimiertes Mengenverhältnis von Glasfüller(n) und Splitterpolymerisat-Füller ermittelt wird, wobei
der Quotient aus dem Wert IG für Texturierung/Plastizität/Konsistenz geteilt durch den Klebrigkeitswert IK als Messgröße zur Gütebeurteilung dient.

2. Verfahren nach einem der vorstehenden Ansprüche, bei dem zusätzlich das Mengenverhältnis zwischen Splitterpolymerisat-Füller und dem/den weiteren Füllstoff(en) variiert wird.

3. Verfahren nach Anspruch 1 oder 2, wobei 0,1 bis 5 Gew.-%, bezogen auf die Gesamtzusammensetzung des Dentalkomposits, des mindestens einen Additivs **A2** der Formel H-Y-Z (I) zugegeben werden.

4. Verfahren nach einem der Ansprüche 1 bis 3, wobei das Additiv **A2** ausgewählt wird aus Wasser, Polyethylenglykol (PEG) mit MG < 250 und Glycerin.

5. Verfahren nach einem der Ansprüche 1 bis 4, wobei das Dentalkomposit folgende Komponenten umfasst:
65 bis 75 Gew.-% Glasfüller
1 bis 5 Gew.-% Splitterpolymerisat-Füller
0,1 bis 5 Gew.-% Additiv **A2** der Formel H-Y-Z
15 bis 25 Gew.-% Monomermischung
15 bis 20 Gew.-% Vernetzer.

6. Fließfähiges, Füllstoffe enthaltendes, durch das Verfahren nach einem der Ansprüche 1 bis 5 behandeltes Dentalkomposit,
wobei die Füllstoffe mindestens einen Glasfüllstoff und mindestens einen Füllstoff **A1** aus der Gruppe der Splitterpolymerisate auf der Basis von gemahlenem, zusammen mit anorganischen Partikeln aus Siliziumdioxid vorpolymerisiertem Polymer umfassen und
wobei das Dentalkomposit mindestens eine hydroxyfunktionalisierte Verbindung der Formel H-Y-Z (I) mit einem Molekulargewicht < 250 g/mol als Additiv **A2** enthält, worin bedeuten:
Y = -O-, -S-, -CO-, -OSi(OR¹)₂-, -OE
Z = H, OH, SH, NH₂, COOH, COOR²
E = -CₙHₘOₚ-
R¹ = H, C₁-C₄-Alkyl
R² = C₁-C₁₅-Alkyl, durch ein oder mehrere O-Atome unterbrochenes C₂-C₁₄-Alkyl,
n = 2 bis 5
m = 4 bis 10
p = n und
wobei die Additive **A1** und **A2** jeweils mit maximal 5 Gew.-%, bezogen auf die Gesamtzusammensetzung des Dentalkomposits, enthalten sind und
wobei das Dentalkomposit einen Quotienten IG/IK von 2-5 aufweist, wobei IG für den Wert für Texturierung/Plastizität/Konsistenz und IK für den Klebrigkeitswert IK stehen.

7. Dentalkomposit nach Anspruch 6 wobei der IG/IK Wert im Bereich von 2 bis 3 liegt.

8. Dentalkomposit-Zusammensetzung erhalten durch das Verfahren nach einem der Ansprüche 1 bis 5 enthaltend
65 bis 75 Gew.-% Glasfüller, vorzugsweise BaAl-Silikatglas (röntgenopakes Dentalglas)
1 bis 5 Gew.-% Splitterpolymerisat-Füller
0,1 bis 5 Gew.-% Additiv der Formel (I) H-Y-Z
15 bis 25 Gew.-% Monomermischung, vorzugsweise Urethan(meth)acrylate
15 bis 20 Gew.-% Vernetzer, bevorzugt aus der Gruppe TCD-di-HEA und HEMA-TMDI, wobei das Dentalkomposit einen Quotienten IG/IK von 2-5 aufweist, wobei IG für den Wert für Texturierung/Plastizität/Konsistenz und IK für den Klebrigkeitswert IK stehen.

9. Verwendung mindestens einer hydroxyfunktionalisierten Verbindung der Formel H-Y-Z (I) mit einem Molekulargewicht < 250 g/mol, worin bedeuten:
Y = -O-, -S-, -CO-, -OSi(OR¹)₂-, -OE
Z = H, OH, SH, NH₂, COOH, COOR²
E = -CₙHₘOₚ-
R¹ = H, C₁-C₄-Alkyl
R² = C₁-C₁₅-Alkyl, durch ein oder mehrere O-Atome unterbrochenes C₂-C₁₄-Alkyl,
n = 2 bis 5
m = 4 bis 10
p = n
als Additiv **A2** und
mindestens ein Splitterpolymerisat-Füller **A1**
jeweils zur Verbesserung der Gebrauchseigenschaften fließfähiger, Füllstoff(e) enthaltender Dentalkomposite, wobei das Additiv **A1** und **A2** jeweils in Mengen von 0,5 bis 5 Gew.-% bezogen auf die Gesamtzusammensetzung, verwendet wird.

## Claims

1. Method for adjusting or improving the usage properties of flowable dental composites containing fillers(s) and glass filler(s), in which
i) at least one prepolymer filler **A1** is added,
ii) the content of component **A1** is varied,
iii) at least one hydroxy-functionalised additive **A2** having a molecular weight < 250 g/mol is added,
the additive **A2** being of formula H-Y-Z (I), where:
Y = -O-, -S-, -CO-, -OSi(OR¹)₂-, -OE
Z = H, OH, SH, NH₂, COOH, COOR²
E = -CₙHₘOₚ-
R¹ = H, C₁-C₄-alkyl
R² = C₁-C₁₅-alkyl, C₂-C₁₄-alkyl interrupted by one or more O-atoms,
n = 2 to 5
m = 4 to 10
p = n,
a maximum of 5 % by weight each of the at least one additive **A1** and **A2,** based on the total composition of the dental composite, being added,
iv) a measurand IK [Force, gram] for adhesiveness and a measurand IG [Force, gram] for texturing or plasticity or consistency are being related to each other,
- the value IG for texturing/plasticity/consistency being determined from the effort of force upon passage through a defined constriction or
from the force opposing a predetermined penetration depth of a measuring stamp, and
- the value IK for adhesiveness being determined from the pull-off force upon pull-off of the measuring stamp from the composite surface, which has been pressed onto the sample with a defined force, and thereafter
v) an optimised proportion of glass filler(s) and prepolymer filler is determined, the quotient of the value IG for texturing/plasticity/consistency divided by the adhesiveness value IK functioning as measurand for quality evaluation.

2. Method according to any one of the preceding claims, in which the proportion of prepolymer filler and the further filler(s) is varied additionally.

3. Method according to claim 1 or 2, 0.1 to 5 % by weight, based on the total composition of the dental composite, of the at least one additive **A2** of formula H-Y-Z (I) being added.

4. Method according to any one of the claims 1 to 3, the additive **A2** being selected from water, polyethylene glycol (PEG) with MW < 250 and glycerol.

5. Method according to any one of the claims 1 to 4, the dental composite comprising the following components:
65 to 75 % by weight glass filler
1 to 5 % by weight prepolymer filler
0.1 to 5 % by weight additive **A2** of formula H-Y-Z
15 to 25 % by weight monomer mixture
15 to 20 % by weight cross-linker.

6. Flowable dental composite containing fillers, treated by a method according to any one of the claims 1 to 5,
the fillers containing at least one glass filler and at least one filler **A1** from the group of prepolymers on the basis of ground polymer pre-polymerised together with inorganic particles from silicon dioxide, and
the dental composite containing at least one hydroxy-functionalised compound of formula H-Y-Z (I) having a molecular weight < 250 g/mol as additive **A2,** where:
Y = -O-, -S-, -CO-, -OSi(OR¹)₂-, -OE
Z = H, OH, SH, NH₂, COOH, COOR²
E = -CₙHₘOₚ-
R¹ = H, C₁-C₄-alkyl
R² = C₁-C₁₅-alkyl, C₂-C₁₄-alkyl interrupted by one or more O-atoms,
n = 2 to 5
m = 4 to 10
p = n, and
the additives **A1** and **A2** each being contained with a maximum of 5 % by weight, based on the total composition of the dental composite, and
the dental composite having a quotient IG/IK of 2-5, IG representing the value for texturing/plasticity/consistency and IK representing the adhesiveness value IK.

7. Dental composite according to claim 6, the IG/IK value being in the range of 2 to 3.

8. Dental composite composition obtained by a method according to any one of the claims 1 to 5 containing
65 to 75 % by weight glass filler, preferably BaAl silicate glass (radiopaque dental glass)
1 to 5 % by weight prepolymer filler
0.1 to 5 % by weight additive of formula (I) H-Y-Z
15 to 25 % by weight monomer mixture, preferably urethane (meth)acrylate
15 to 20 % by weight cross-linker, preferably from the group TCD-di-HEA and HEMA-TMDI,
the dental composite having a quotient IG/IK of 2-5, IG representing the value for texturing/plasticity/consistency and IK representing the adhesiveness value IK.

9. Use of a hydroxy-functionalised compound of formula H-Y-Z (I) having a molecular weight < 250 g/mol, where:
Y = -O-, -S-, -CO-, -OSi(OR¹)₂-, -OE
Z = H, OH, SH, NH₂, COOH, COOR²
E = -CₙHₘOₚ-
R¹ = H, C₁-C₄-alkyl
R² = C₁-C₁₅-alkyl, C₂-C₁₄-alkyl interrupted by one or more O-atoms,
n = 2 to 5
m = 4 to 10
p = n
as additive **A2,** and
at least one prepolymer filler **A1**
each for improving the usage properties of flowable dental composites containing filler(s), the additive **A1** and **A2** each being used in amounts of 0.5 to 5 % by weight, based on the total composition.

## Revendications

1. Procédé pour ajuster ou améliorer les propriétés d'utilisation des composites dentaires fluides contenant de la/des charge(s) et de la/des charge(s) de verre, dans lequel
i) au moins une charge de prépolymère **A1** est ajoutée,
ii) le teneur du composant **A1** est varié,
iii) au moins un additif fonctionnalisé hydroxy **A2** ayant un poids moléculaire
< 250 g/mol est ajouté,
l'additif **A2** étant de la formule H-Y-Z (I), où :
Y = -O-, -S-, -CO-, -OSi(OR¹)₂-, -OE
Z = H, OH, SH, NH₂, COOH, COOR²
E = -CₙHₘOₚ-
R¹ = H, C₁-C₄-alkyle
R² = C₁-C₁₅-alkyle, C₂-C₁₄-alkyle interrompu par un ou plusieurs O-atomes,
n = 2 à 5
m = 4 à 10
p = n,
un maximum de 5 % en poids à chaque fois d'au moins un additif **A1** et **A2,** basé sur la composition totale du composite dentaire, étant ajouté,
iv) un mesurande IK [Force, gramme] pour l'adhésivité et un mesurande IG [Force, gramme] pour la texturation ou la plasticité ou la consistance sont être liés l'un à l'autre,
- la valeur IG pour la texturation/plasticité/consistance étant déterminée de l'effort de force lors du passage à travers d'une constriction définie ou
de la force opposante une profondeur de pénétration prédéterminée d'un poinçon de mesure, et
- la valeur IK pour l'adhésivité étant déterminée de la force de traction lors de la traction du poinçon de mesure loin de la surface de composite, qui a été pressé sur l'échantillon avec une force définie, et ensuite
v) une proportion optimisée de la/des charge(s) de verre et du prépolymère est déterminée,
le quotient de la valeur IG pour la texturation/plasticité/consistance divisé par la valeur d'adhésivité IK servant du mesurande pour l'évaluation de qualité.

2. Procédé selon l'une des revendications précédentes, dans lequel la proportion de la charge de prépolymère et l'/les autre(s) charge(s) est variée additionnellement.

3. Procédé selon la revendication 1 ou 2, 0,1 to 5 % en poids, basés sur la composition totale du composite dentaire, de l'au moins un additif **A2** de la formule H-Y-Z (I) étant ajoutés.

4. Procédé selon l'une des revendications 1 à 3, l'additif **A2** étant sélectionné parmi de l'eau, du polyéthylène glycol (PEG) avec PM < 250 et glycérine.

5. Procédé selon l'une des revendications 1 à 4, le composite dentaire comprenant les composants suivants :
65 à 75 % en poids de la charge de verre,
1 à 5 % en poids de la charge de prépolymère
0.1 à 5 % en poids de l'additif **A2** de la formule H-Y-Z
15 à 25 % en poids de la mixture monomère
15 à 20 % en poids de l'agent de réticulation.

6. Composite dentaire fluide contenant des charges, traité par le procédé selon l'une des revendications 1 à 5,
les charges contenant au moins une charge de verre et au moins une charge **A1** de la groupe des prépolymères sur la base du polymère moulu prépolymérisé conjointement avec des particules inorganiques du dioxyde de silicium, et
le composite dentaire contenant au moins un composé fonctionnalisé hydroxy de la formule H-Y-Z (I) ayant un poids moléculaire < 250 g/mol en tant que l'additif **A2,** où :
Y = -O-, -S-, -CO-, -OSi(OR¹)₂-, -OE
Z = H, OH, SH, NH₂, COOH, COOR²
E = -CₙHₘOₚ-
R¹ = H, C₁-C₄-alkyle
R² = C₁-C₁₅-alkyle, C₂-C₁₄-alkyle interrompu par un ou plusieurs O-atomes,
n = 2 à 5
m = 4 à 10
p = n, et
les additifs **A1** et **A2** à chaque fois étant contenus avec un maximum de 5 % en poids, basé sur la composition totale du composite dentaire, et
le composite dentaire ayant un quotient IG/IK de 2-5, IG représentant la valeur pour la texturation/plasticité/consistance et IK représentant la valeur d'adhésivité IK.

7. Composite dentaire selon la revendication 6, le valeur IG/IK étant de l'ordre de 2 à 3.

8. Composition de composite dentaire obtenu par le procédé selon l'une des revendications 1 à 5 contenant
65 à 75 % en poids de la charge de verre, de préférence du verre au silicate de BaAl (de la verre dentaire radio-opaque)
1 à 5 % en poids de la charge de prépolymère
0.1 à 5 % en poids de l'additif de la formule (I) H-Y-Z
15 à 25 % en poids de la mixture monomère, de préférence du (méth)acrylate d'uréthane
15 à 20 % en poids de l'agent de réticulation, de préférence de la groupe TCD-di-HEA et HEMA-TMDI,
le composite dentaire ayant un quotient IG/IK de 2-5, IG représentant la valeur pour la texturation/plasticité/consistance et IK représentant la valeur d'adhésivité IK.

9. Utilisation d'un composé fonctionnalisé hydroxy de la formule H-Y-Z (I) ayant un poids moléculaire < 250 g/mol, où :
Y = -O-, -S-, -CO-, -OSi(OR¹)₂-, -OE
Z = H, OH, SH, NH₂, COOH, COOR²
E = -CₙHₘOₚ-
R¹ = H, C₁-C₄-alkyle
R² = C₁-C₁₅-alkyle, C₂-C₁₄-alkyle interrompu par un ou plusieurs O-atomes,
n = 2 à 5
m = 4 à 10
p = n, et
en tant que l'additif **A2,** et
au moins une charge de prépolymère **A1**
à chaque fois pour améliorer les propriétés d'utilisation des composites dentaires fluides contenant de la/des charge(s), l'additifs **A1** et **A2** à chaque fois étant utilisé en montants de 0,5 à 5 % en poids, basés sur la composition totale.
